# EUROPEAN PATENT SPECIFICATION

(11) **EP 0 341 053 B1**
(45) Date of publication and mention of the grant of the patent: **15.12.1993**
(21) Application number: 89304482.6
(22) Date of filing: 04.05.1989
(51) Int. Cl.: C07D 233/14, B01F 17/32, C09D 11/10

(54) **Semi-polar hyperdispersants for pigment bases**
Semi-polare Hyperdispergiermittel für Pigmentbasen
Hyperdispersants semi-polaires pour bases de pigment

(30) Priority: 05.05.1988 US 190623
(43) Date of publication of application: 08.11.1989
(73) Proprietor: SUN CHEMICAL CORPORATION, Fort Lee New Jersey 07026 (US)
(72) Inventor: Kveglis, Albert A., Pine Brook New Jersey (US); Gruben, Arnold H., Cedar Grove New Jersey (US)
(74) Representative: Lawrence, Peter Robin Broughton

(56) References cited:
- US-A- 3 235 560
- US-A- 3 717 447

## Description

This invention relates to quaternized oligourethanes which are useful as dispersing agents in preparing high-solids pigment concentrates for use in the manufacture of inks, especially inks intended for use in gravure and packaging applications.

Due to the economics of the ink business today, it is highly desirable to be able to process pigments into high-solids concentrates. Typically, such concentrates would contain from 30 to 60% pigment. Ideally, they could be let down in a wide variety of vehicles in order to produce a wide range of finished inks.

The economic advantages offered by such concentrates are twofold. First, they would greatly reduce the inventory of intermediates which must be carried by an ink plant. Only one intermediate dispersion would be needed for each pigment versus the several which are now typically used. Second, energy consumption would be considerably reduced. The higher the pigment concentration at which dispersion can be effected, the less the total volume of material that must be processed through a mill and therefore the less total energy consumed in processing the pigment.

Traditionally, vehicles for lithographic inks are composed of alkyd resins and/or rosin derivatives and/or hydrocarbon resins, together with high-boiling hydrocarbon solvents. It is known in the art that satisfactory dispersions containing 30 to 60% pigment cannot be prepared using such traditional vehicle components alone. Usually mixtures at such solids levels cannot be processed in standard milling equipment. In the few cases where they can be processed, they yield dispersions with unacceptable rheological properties, i.e. dispersions which cannot be pumped or otherwise conveniently transferred from one vessel to another. Such dispersions usually also show inferior color development and poor aging stability.

Recently attempts have been made to overcome the above-described problems by using compounds which are better dispersants for pigments than are the traditional alkyd resins and rosin derivatives. For example U. S. Pat. No. 4,224,212 describes the use of dispersing agents attained by reacting a poly (lower alkylene) imine with a polyester having free carboxylic acid groups to form reaction products containing at least two polyester chains attached to each poly (lower alkylene) imine chain. Preferred polyesters are the polyesters of an hydroxy carboxylic acid of the formula HO-R-COOH where R is a divalent aliphatic radical containing at least 8 carbon atoms in which there are at least 4 carbon atoms between the hydroxy and carboxylic acid groups. Preferred polyesters are also the polyesters formed from a mixture of aforementioned hydroxy carboxylic acids with a carboxylic acid which is free from hydroxy groups.

U. S. Patent No. 4,415,705 describes the use of rosin derivatives as dispersants. These dispersants are attained by reacting a poly (lower alkylene) imine having a molecular weight of 1,000 to 15,000 with a polyester obtained by esterifying hydroxy stearic acid or its oligomer, with tall oil rosin. This patent states that such dispersants have been found to be superior to products made from wood rosin or gum rosin.

US 3717447 relates to a group of oligourethane compounds for use as additivies in hydrocarbon fuel. The patent discloses carbonates but does not disclose any compounds in which the nitrogen of the carbonate group is substituted with other organic groups.

US 3235560 discloses substituted 1,3-imidazolines which have useful fluorescent properties, for use as dyes. The compounds disclosed are polyesters.

A novel class of quaternized oligourethanes has been discovered. These quaternized oligourethanes have been found to be very useful in preparing non-flocculating dispersions of high loadings of inorganic or organic pigments or dyestuffs. Such dispersions have been found to produce inks which yield excellent results especially when such inks are utilized for gravure and packaging applications.

The novel quaternized oligourethanes of the present invention may be represented by the general formula 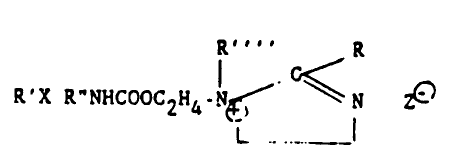
wherein X represents the radical -CONH- or -OCONH- such that when X is -CONH-, R' is an ester resulting from the reaction of a C₂-C₁₈ hydroxyalkanoic acid or a C₃ - C₁₈ hydroxyalkenoic acid and rosin selected from wood rosin, gum rosin. and tall oil rosin, and when X is -OCONH-, R' is a C₃-C₁₈ linear or branched alkyl, aryl, alkaryl, aralkyl, cycloalkyl or the radical 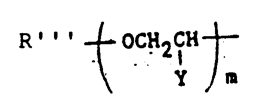
wherein Y is hydrogen or methyl, R''' is a C₁-C₆ alkyl and m is an integer of 1 to 20;
R is a C₁₁ - C₁₇ linear or branched alkyl, alkenyl, alkynyl or cycloalkyl;
R'' is a C₆ - C₁₄ linear or branched alkyl, aryl, alkaryl, aralkyl or cycloalkyl or a dimer or trimer thereof;
R'''' is hydrogen or a C₁ - C₄ linear or branched alkyl; and
Z is R''''OSO₃, R'''''SO₃, Cl, Br, I, NO₃ or
(R'''')₃₋ₙHₙPO₄ or (R'''')₃₋ₙHₙPO₃
wherein n is an integer of 0 to 3, and R''''' is aryl, CH₃ or CF₃.

The rosin which is used for reaction with hydroxyalkanoic acid or hydroxyalkenoic acid may be wood rosin, gum rosin or tall oil rosin, but tall oil rosin is preferred.

Preferably, in the general formula given above, X is a urethane radical i.e. -OCONH-. Preferably the ester is that which results from the reaction of a C₆ - C₁₈ hydroxy alkanoic acid with tall oil rosin.

In the above general formula, it is preferred that R′ be the radical 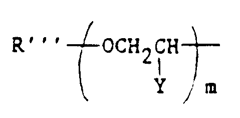
wherein Y is hydrogen or methyl, R''' is a C₁ - C₆ alkyl, preferably a C₂ - C₄ alkyl and m is an integer of 1 to 20, preferably 2 to 10. It is also preferred that R˝ be a C₇ - C₁₃ aryl and Z be R''''OSO₃.

The quaternized oligourethanes are excellent dispersing agents for dispersing solid materials in organic liquids. The dispersion can be obtained by any of the conventional and well known methods of preparing dispersions. Thus the solid, the organic liquid and the dispersing agent may be mixed in any order and the mixture then subjected to a mechanical treatment to reduce the particle size of the solid, for example by ball milling, bead milling, gravel milling or plastic milling until the dispersion is formed.

Alternatively, the solid can be treated to reduce its particle size independently or in admixture with either the organic liquid or the dispersing agent, and the other ingredient or ingredients then added following which dispersion can be obtained by stirring the mixture. A dispersion obtained in this way and comprising the solid in finely divided form and one or more dispersing agents is a further feature of this invention.

The amount of dispersing agent present in the dispersion is generally in the range of 1 - 20 wt. %, based on the weight of the dispersion. The dispersion generally contains 30 - 75 wt. % of the solid material and 10 - 50 wt. % of the organic liquid, based on the weight of the dispersion. It may also be helpful to incorporate up to 5 wt. %, based on the weight of the dispersion, of a nonionic surfactant such as sorbitol monooleate, glycerolmonooleate, polyethylene glycol mono nonyl phenyl ether, poly(ethylene-co-propylene)glycol mono octyl phenyl ether, ethoxylated sorbitan esters, long chain fatty acid esters of polyethyleneglycol, lecithin, tertiary acetylenic diols, etc.

The solid may be any particulate solid material of an inorganic or organic nature which is substantially insoluble in the organic liquid at the desired temperature of usage and which is capable of comminution into a finely divided form. This invention is of particular value when the solid is a pigment or dyestuff. For the purposes of this invention, the term "pigment" includes both inorganic and organic pigments and also lakes and toners.

As examples of organic pigments there may be mentioned azo, thionindigo, anthraquinone, anthanthrone and isodibenzanthrone pigments, vat dye pigments, triphenodioxazine pigments, phthalocyanine pigments for example copper phthalocyanine, its nuclear chlorinated derivatives and copper tetraphenyl or octaphenyl phthalocyanine and other heterocyclic pigments, for example linear quinacridone.

As examples of inorganic pigments there may be mentioned chrome pigments including the chromates of lead, zinc, barium and calcium and various mixtures and modifications such as are commercially available as pigments of greenish-yellow to red shades under the names primrose, lemon, middle orange, scarlet and red chromes. Modified chrome pigments may contain for example sulphate radicals and/or additional metals such as aluminium, molybdenum and tin. Further examples of inorganic pigments are carbon black, titanium dioxide, zinc oxide, Prussian blue and its mixtures with chrome yellows which are known as Brunswick Greens or chrome greens, cadmium sulphide and sulphoselenide, iron oxides, vermilion and ultramarine. These and various other pigments suitable for use in the present invention are described in Volume 2 of "Colour Index 6 2nd Edition," published jointly in 1956 by the Society of Dyers and Colourists and the American Association of Textile Chemists and Colourists, under the heading of "Pigments" and in subsequent authorized amendments thereto.

The term "lake" denotes a water-insoluble metal salt or complex of an organic dyestuff which has been precipitated on a water-insoluble inorganic substrate such as alumina.

The term "toner" denotes a water-insoluble metal salt or complex, in particular a calcium or barium salt or complex, of a soluble or sparingly soluble organic dyestuff, in particular an azo dyestuff, which has optionally been prepared in the presence of an extender such as rosin.

As specific examples of the said lakes and toners there may be mentioned the barium toner of 1-(2'-sulpho-4'-methyl-5'-chlorophenylazo)-2-hydroxy-3-naphthoic acid, the nickel complex of 3-(4'-chlorophenylazo)-quinoline-2,4-diol, the rosinated barium toner of 1-(2'-sulpho-4'-chloro-5'-methylphenylazo)-2-naphthol, the aluminium lake of 1,4-dihydroxyanthraquinone-2-sulphonic acid and, above all, a rosinated calcium toner of 1-(2'-sulpho-4'-methylphenylazo)-2-hydroxy-3-naphthoic acid.

Especially preferred pigments for use in the present invention are those typically employed for gravure and packaging ink systems such as diarylide yellow, BON red, carbon black, Red Lake C, Lithol Rubine, phthalocyanine blue, phthalocyanine green, molybdenum orange and titanium dioxide.

Dyestuffs which are useful in the present invention are those which are water soluble or water-insoluble such as basic, acid and direct dyestuffs. The dyestuffs include azo types such as monoazo and diazo and metal derivatives thereof, anthraquinone, nitro, phthalocyanine, methine, styryl, naphthoperinone, quinaphthalone, diarylmethane, triarylmethane, xanthine, azine, oxazine and thiazine dyestuffs. If desired the dyestuffs can be reactive dyestuffs which contain groups capable of forming covalent bonds with textile materials.

Any organic liquid may be used in the dispersion but hydrocarbons are preferred. As examples of such liquids there are mentioned aromatic hydrocarbons such as benzene, toluene, xylene, aliphatic and cycloaliphatic hydrocarbons such as petroleum fractions, white spirit and cyclohexane, and high boiling mineral oils such as spindle oil. Alternative organic liquids are halogen substituted hydrocarbons such as chlorobenzene, trichloroethylene, perchloroethylene, 1,1,1-trichloroethane, methylene dichloride, chloroform, 1,1,2-trichloro-1,2,2-trifluoroethane, carbon tetrachloride, tetrachloroethane or dibromoethylene and mixtures of these compounds, esters such as ethyl acetate, propyl acetate and butyl acetate and heat bodied linseed oils used as lithographic varnish media and ketones such as methylethylketone methylisobutyl ketone and cyclohexanone. Mixtures of such solvents may be used. The solvents may contain other materials in solution, for example the alkyd, nitrocellulose, acrylic, urea/formaldehyde, melamine/formaldehyde or other resins used in paint media or zinc/calcium rosinates used in gravure ink media. Especially preferred solvents are aliphatic hydrocarbons which are compatible with gravure and packaging ink systems, such as hexanes, heptanes, octanes, cyclohexane, methylcyclohexane, lactol spirits, naphtha and mineral spirits.

The dispersions of this invention are fluid or semi-fluid compositions containing the solid in finely divided and usually deflocculated form, and can be used for any purpose for which dispersions of these particular solids are conventionally used. Thus the pigment dispersions are of value in the manufacture of printing inks particularly publication gravure and packaging inks by incorporating the dispersions with the other components conventionally used in the manufacture of such inks. Typically such inks will contain a pigment dispersed in an ink vehicle such as a liquid petroleum hydrocarbon solution of the ink, plus resin and 1 - 20 wt% of the quaternized oligourethane of this invention. These dispersions are also of value in the manufacture of paints, for which purpose the dispersions are incorporated into conventional alkyd or other resins.

The dyestuff dispersions are useful in the preparation of textile printing inks or solvent dyeing systems and particularly where the dyestuff is a sublimable disperse dyestuff useful in transfer printing. Inks and paints containing such dispersions are further features of the present invention.

The process for preparing the novel quaternized oligourethanes of this invention will depend on whether it is desired to make a product wherein X in the general formula above is to be the amide radical -CONH- or the urethane radical -OCONH-. If a product is desired with the amide radical, then the 3-step process outlined below is followed. If a product containing the urethane radical is desired, then the 2-step process outlined below is followed.

The 3-step process involves:
(a) reacting a C₂ - C₁₈ hydroxyalkanoic acid or a C₃ - C₁₈ hydroxyalkenoic acid with rosin in the presence of an esterification catalyst;
(b) reacting the ester resulting from step (a) with a 1-hydroxyethyl-2-C₁₁-C₁₇ imidazoline and a C₆-C₁₄ polyisocyanate;
(c) quaternizing the oligourethane resulting from step (b) by reacting it with a C₁ - C₄ linear or branched alkyl halide, alkyl nitrate, alkyl phosphate, alkyl phosphite, alkyl sulfate, aryl sulfonate, alkyl sulfonate or a mineral acid or halogen acid.
The acid may be a C₃ - C₁₈ hydroxyalkenoic acid such as 4-hydroxybutenoic acid, 4-hydroxy-3-pentenoic acid, castor fatty acid 6-hydroxy-3-hexenoic acid and the like, but is preferably a C₆ - C₁₈ hydroxyalkanoic acid such as commercial 12-hydroxystearic acid, w-hydroxycaproic acid, glycolic acid, 4-hydroxybutyric acid, 10-hydroxydecanoic acid, 2-hydroxyisocaproic acid and the like are also useful. As for the rosin, it is preferably tall oil rosin, although wood rosin or gum rosin are also useful.

The esterification reaction is carried out in the presence of an esterification catalyst generally at elevated temperatures in the range of about 170 to 210°C for about 8 to 18 hours. Useful esterification catalysts include dibutyltin oxide, tetrabutyl titanate, triphenylphosphite, p-toluenesulfonic acid, sulfuric acid, etc. Typically, the ratio of acid to rosin will be in the range of 1 to 6 moles of acid per mole of rosin. It is preferred that the relative proportions of acid and rosin be selected such that each oligomer molecule will contain up to one carboxyl group and the degree of esterification is between n - 1 to n - 6. The resultant carboxyl-terminated polyester will have a typical acid value of 70 + 5 mg KOH/g sample.

In step (b), 0.2 - 0.8 mole of ester from step (a) is reacted with .8 - 0.2 mole of the imidazoline as well as with 0.8 - 1.0 mole of polyisocyanate. Desirably, this reaction is carried out in the presence of a catalyst such as stannous octoate. Preferred hydroxyimidazolines include
1-hydroxyethyl-2-heptadecenyl imidazoline,
1-hydroxyethyl-2-heptadecyl imidazoline,
1-hydroxyethyl-2-pentadecyl imidazoline,
1-hydroxyethyl-2-tridecylimidazoline and the like. Preferred polyisocyanates include mixed 2,6- and 2,4-tolylene diisocyanates, tris(4-isocyanatophenyl)methane, 4,4'-diisocyanatodiphenylmethane, hexamethylenediisocyanate, 4,4'-diisocyanatodicyclohexylmethane, phenyldiisocyanate, and the like. The reaction mixture of step (b) is maintained at a temperature of 95 to 100°C for 2 to 5 hours.

The oligourethane resulting from step (b) is then quaternized in step (c). Quaternization is desirable since it introduces a polar or ionic cluster to one end of the non-polar chain of the oligourethane. The polar (i.e. ionic) end attaches to the pigment particle while the non-polar end serves to sterically repel other similarly-coated particles, thus preventing aggregation (or flocculation) while at the same time permitting high loadings of pigment in the dispersion. Generally the quaternizing reagent mentioned above will be utilized in a molar ratio of 0.95 to 1.0 mole per mole of tertiary nitrogen in the oligourethane. Suitable quaternizing agents include C₁ - C₄ linear or branched alkyl sulfates, phosphates, phosphites, halides or nitrates, phosphoric acid, nitric acid, hydrochloric acid, hydrobromic acid, hydroiodic acid, p-toluenesulfonic acid and sulfuric acid esters (which are particularly preferred).

The two-step process resulting in an oligourethane containing the urethane radical is as follows:
(a) reacting a C₃ - C₁₈ linear or branched alkyl, aryl, alkaryl, aralkyl, cycloalkyl mononofunctional alcohol or a monofunctional alcohol containing the radical 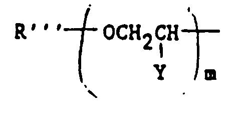 with a 1-hydroxyethyl-2-C₁₁-C₁₇ imidazoline and a C₆-C₁₄ polyisocyanate; and
(b) quaternizing the oligourethane resulting from step (a) by reacting it with a C₁ - C₄ linear or branched alkyl halide, alkyl nitrate, alkyl phosphate, alkyl phosphite alkyl sulfate, aryl sulfonate, alkyl sulfonate or a mineral acid or halogen acid.

Suitable examples of alcohols to be employed in step (a) include hydroabietyl alcohol, butoxytriglycol and isostearyl alcohol. The hydroxyimidazolines and polyisocyanates are the same as those indicated above for step (b) in the 3-step process described above. The molar ratios of alcohol to hydroxyimidazoline and polyisocyanate are the same as in the case of the molar ratios of the ester to hydroxyisidazoline and the polyisocyanate; the molar ratio range of quaternizing agent to moles of tertiary nitrogen in the oligourethane is the same irrespective of whether the oligourethane is prepared by the 3-step process or the 2-step process.

This invention is illustrated but not limited by the following Examples in which the parts and percentages are by weight:

### EXAMPLE 1

### STEP A

Into a 4-neck reaction flask were charged 75.5 g 12-hydroxystearic acid, 24.4 g tall oil rosin and 0.1 g dibutyl tin oxide. A nitrogen blanket was maintained over the reaction mixture which was heated to 200°C. with agitation. The water of reaction was removed using a Dean and Stark trap and the heating with agitation was continued at 200 - 205°C. for about 10 hours until an ester having an acid value of about 70 mg KOH/g was obtained.

### STEP B

To 42.5g of the ester obtained from step A were added 19.2g 1-hydroxyethyl-2-heptadecenyl imidazoline, 0.08 g stannous octoate and 30 g of heptane solvent. The reactants were warmed to reflux temperature with agitation while continuing the nitrogen blanket. When a solution resulted, it was heated to 95 - 100°C. and 5.5 g tolylene diisocyanate gradually added, while maintaining the temperature at about 100°C. The reaction was complete after about 3 hours as indicated by the absence of the isocyanate peak at 4.45 microns under infrared spectrophotometry. The amine value of the resulting oligourethane was then measured to determine the required amount of quaternization agent.

### STEP C

The oligourethane obtained in step B was quaternized with 2.8 g of diethyl sulfate. The quaternization reaction was carried out, with agitation and a nitrogen blanket, at a temperature of 95 - 100°C. with the diethyl sulfate added over about a two hour period. An amine value of 1.0 mg KOH/g indicated completion of the reaction. The product was discharged upon cooling to about 45°C. and was strained through a nylon bag-type filter. The product had a viscosity of about 50 poises at 25°C. by Gardner tube, a varnish standard color of 12, a solids content of 70% N.V. in heptane and a specific gravity of 0.89.

### EXAMPLE 2

EXAMPLE 1 was repeated with a modification in that polyethylene glycol 200 was also added to the reactants in step B. The reaction mixture for step B was as follows:

| | |
|---|---|
| Example I - step A ester | 28.2 g |
| oleyl imidazoline | 25.5 g |
| polyethylene glycol 200 | 2.1 g |
| stannous octoate | 0.1 g |
| heptane solvent | 30.0 g |

The reactants listed above were heated and agitated in the same manner as in Example 1 and 7.7 g tolyl diisocyanate were gradually added, while maintaining the temperature at about 100°C. In step C, quaternization required 6.4 g of diethyl sulfate indicating a higher degree of quaternization in the final product.

### EXAMPLE 3

15.3 parts of the quaternized oligourethane solution from Step c) of Example 1 were dispersed in an Eiger mill with 27.7 parts heptane, 7.0 parts n-butyl alcohol and 50.0 parts of cyan blue pigment. The dispersion particle size was too fine to be measured on a grind gauge and was of the order of 0.2 - 0.3 micrometers. 18.5 parts of this dispersion were mixed for 5 minutes in an Ultra Turrax Mill with 9.8 parts of polyvinylchloride copolymer resin and 7.5 parts polyester plasticizer, 1.0 part wax, 48.2 parts n-propylacetate and 15.0 parts isopropylacetate. The resultant ink, after being printed on a polyester film, showed a much stronger color development than a comparable ink at the same pigment level but which did not contain the quaternized oligourethane of the present solution.

### EXAMPLE 4

In this example, an alcohol was employed as the starting material for the 2-step process in order to obtain a quaternized oligourethane that contained the urethane radical rather than the amide radical. 15.48 parts of hydroabiethyl alcohol and 6.1 parts butoxytriglycol were mixed with 25.8 parts of 1-hydroxyethyl-2-heptadecenylimidazoline, 0.1 part stannous octoate and 29.9 parts reagent grade n-heptane in a 4-neck round bottom flask equipped with mechanical agitator, thermometer, dry N₂ inlet, reflux condenser and addition funnel. The mixture was heated under N₂ to 95 - 100°C. for solution. Toluene diisocyanate, 12.208 parts, was then added dropwise at a rate so as to maintain reflux at about 100°C. After 3 - 4 hours reaction at 95 - 100°C no IR NCO absorption peak was observed at 4.45 micrometers.

10.409 parts 98% diethyl sulfate was then added dropwise over one hour at 95 - 100°C. After 1 - 2 hours at 95 - 100°C. an amine titration of 1.0 mg KOH/g indicated that quaternization was essentially complete. The solution was cooled to 40 - 50°C. and strained through a nylon organdy bag filter. The solution had the following properties:

| | |
|---|---|
| Viscosity: | 200 - 300 poises @ 25°C. |
| Color: | 11 Gardner |
| Solids: | 70 + 1% |

The quaternized oligourethane solution prepared as above (13.2 parts) was dispersed with 37.8 parts heptane, 6.0 parts n-butyl alcohol and 43.0 parts 2B red pigment in an Eiger Mill to give a dispersion with pigment particle size between 0.2 - 0.3 micrometers. The dispersion was too fine to measure on a grind gauge.

The resultant red 2B dispersion (20.3 parts) was blended for 5 minutes with 28.8 parts maleic resin, 9.0 parts ethylacetate, 23.4 parts ethanol, 13.5 parts of a 35% solution of nitrocellulose and 5.0 parts wax in the Ultra Turrax Mill. The resultant ink after printing on uncoated bleached paper stock showed better color development at the same pigmentation level as an ink which did not contain the quaternized oligourethane.

### EXAMPLE 5

The quaternized oligourethane solution prepared in accordance with Example 4 (10.5 parts) was dispersed in an Eiger Mill with 50.4 parts heptane, 4.8 parts n-butyl alcohol and 34.3 parts diarylide yellow pigment. The dispersion was too fine to be measured on a grind gauge and was in the range of 0.2 - 0.3 micrometer particle size.

The diarylide yellow pigment dispersion (24. 5 parts) was mixed with 8.1 parts n-propyl alcohol, 23.0 parts isopropyl alcohol, 24.4 parts polyamide resin, 5.0 parts naphtha, 8.0 parts maleic resin, 4.0 parts wax and 3.0 parts water in an Ultra Turrax Mill in the same manner as above. The resultant ink, when printed on high slip polyethylene and polypropylene films, showed better heat resistance, better gloss and better color strength versus the same ink made at the same pigmentation level without the quaternized oligourethane of the present invention.

### EXAMPLE 6

The same apparatus as described in Example 4 was employed in this example. 28.59 parts 1-hydroxyethyl-2-heptadecenylimidazoline, 16.87 parts butoxytriglycol, 0.1 part stannous octoate and 29.90 parts heptane were mixed. The mixture was heated to 95°C. under a nitrogen blanket and thereafter dropwise addition of 12.81 parts toluene diisocyanate was begun. The rate was adjusted to maintain the temperature below 100°C, and the urethanation reaction was completed in about 3 hours as noted by the absence of the NCO peak at 4.5 microns as determined on an analytical infrared spectrophotometer.

Thereafter, 11.73 parts 98% diethyl sulfate were added over one hour at 95 - 100°C. After 1 - 2 hours at 95 - 100°C., an amine value titration of less than 1.0 mg KOH/g indicated that quaternization was complete. The product was cooled to 45 - 50°C. and filtered through a nylon bag filter. It had the following properties:

| | |
|---|---|
| Viscosity: | 130 - 200 poises at 25°C. |
| Color: | 12 Gardner |
| Solids: | 70 + 1% |

### EXAMPLE 7

This example employed the same apparatus and general procedure as described in the preceding examples. A quaternized oligourethane solution was prepared from 27.04 parts 1-hydroxyethyl-2-heptadecenyl imidazoline, 6.4 parts butoxytriglycol, 13.69 parts isostearyl alcohol, 0.1 parts stannous octoate, 29.9 parts heptane, 12.12 parts toluene diisocyanate and 10.75 parts 98% diethyl sulfate. After the product was cooled to 45 - 50°C. and cast through a nylon bag filter, it had the following properties:

| | |
|---|---|
| Viscosity: | 120 - 200 poises at 25°C. |
| Color: | 11 Gardner |
| Solids: | 70 + 1% |

### EXAMPLE 8

The procedure of Example 7 was repeated in order to obtain a quaternized oligourethane solution based on 21.75 parts 1-hydroxyethyl-2-heptadecenyl imidazoline, 27.53 parts isostearyl alcohol, 0.1 part stannous octoate, 29.9 parts n-heptane, 12.18 parts toluene diisocyanate and 8.54 parts 98% diethyl sulfate. The product after cooling exhibited the following properties:

| | |
|---|---|
| Viscosity: | 30 - 50 poises at 25°C. |
| Color: | 9 Gardner |
| Solids: | 70 + 1% |

### EXAMPLE 9

This example was carried out in order to provide material for comparative purposes and its use is shown in the table below. Using the same type of equipment as described in Example 4, 75.5 parts 12-hydroxystearic acid, 24.4 parts tall oil rosin and 0.1 part dibutyltin oxide were mixed. The mixture was heated to 200°C. and held at that point for 9 - 12 hours until an acid value of 69 - 71 mg KOH/g was obtained. To 93.5 parts of this reaction product maintained at 70°C. were added 6.5 parts polyethyleneimine. The temperature of the mixture was slowly raised to 150°C. at which point a slight exothermic reaction occurred. The reaction mixture was held an additional 4 - 6 hours at this temperature until an acid value of 50 - 55 mg KOH/g was obtained. Thereafter, the molten oligomer was cooled to 120°C. and heptane was slowly added under reflux to provide a 70% nonvolatile content solution. The solution was then cooled to 40 - 50°C. cast through a nylon organdy bag filter and such solution exhibited the following properties:

| | |
|---|---|
| Viscosity: | 5 - 10 poises at 25°C. |
| Color: | 14 - 15 Gardner |
| Solids: | 70 + 1% |
| Acid Value: | 52.0 (on solids) |

### EXAMPLE 10

In this example, various quaternized oligourethane solutions were utilized to provide the dispersions indicated in the table below. The quaternized oligourethane solutions were utilized as 70% solids solution in heptane.

**TABLE I**

| | | | | | | |
|---|---|---|---|---|---|---|
| Quaternized Oligourethane Source | Ex.1 | Ex.1 | Ex.9 | Ex.6 | Ex.7 | Ex.8 |
| Quaternized Oligourethane Amount | 16.3 | 13.4 | 12.6 | 15.3 | 15.3 | 15.3 |
| Red Lake C | -- | 44.0 | 41.3 | -- | -- | -- |
| Carbon Black | 50.0 | -- | -- | -- | -- | -- |
| Bon Pigment | -- | -- | -- | 50.0 | -- | -- |
| Bon Red Pigment | -- | -- | -- | -- | -- | 50.0 |
| Cyan Blue | -- | -- | -- | -- | 50.0 | -- |
| Heptane | 26.7 | 36.6 | 40.3 | 27.7 | 27.7 | 27.7 |
| n-Butyl Alcohol | 7.0 | 6.0 | 5.8 | 7.0 | 7.0 | 7.0 |

As is evident from the table indicated above, the quaternized oligourethane solution of Example 1 allowed a higher Red Lake C pigment loading versus comparative Example 9, while still maintaining acceptable viscosity. The table above further indicates the benefit of obtaining free-flowing dispersions at relatively high loadings of pigment, thereby presenting the ink manufacturer and user with wide formulation latitude.

## Claims (Claims for the following Contracting State(s): AT, BE, CH, DE, FR, GB, GR, IT, LI, LU, NL, SE)

1. A quaternized oligourethane having the general formula: 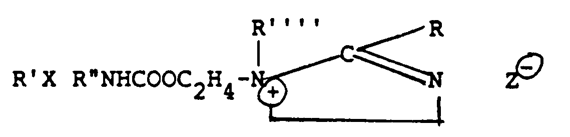 wherein X represents the radical -CONH- or -OCONH- such that when X is -CONH-, R' is an ester resulting from the reaction of a C₂-C₁₈ hydroxyalkanoic acid or a C₃ - C₁₈ hydroxyalkenoic acid and rosin, selected from wood rosin, gum rosin, or tall oil rosin, and when X is -OCONH-, R' is a C₃-C₁₈ linear or branched alkyl, aryl, alkaryl, aralkyl, cycloalkyl or the radical 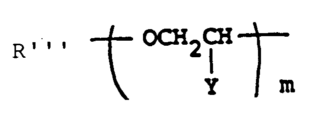 wherein Y is a hydrogen or methyl, R''' is a C₁-C₆ alkyl and m is an integer of 1 to 20;
R is a C₁₁ - C₁₇ linear or branched alkyl, alkenyl, alkynyl or cycloalkyl;
R'' is a C₆ - C₁₄ linear or branched alkyl, aryl, alkaryl, aralkyl or cycloalkyl or a dimer or trimer thereof;
R'''' is hydrogen or a C₁ - C₄ linear or branched alkyl;
and
Z is R''''OSO₃, Cl, Br, I, NO₃, R'''''SO₃,
(R'''')₃₋ₙHₙPO₄ or (R'''')₃₋ₙHₙPO₃
wherein n is an integer of 0 to 3 and R''''' is aryl, CH₃ or CF₃.

2. The quaternized oligourethane of claim 1 wherein the rosin is tall oil rosin.

3. The quaternized oligourethane of claim 1 wherein X is the radical -OCONH-.

4. A printing ink comprising pigment dispersed in an ink vehicle comprised of a liquid petroleum hydrocarbon solution of an ink, resin and 1 - 20 wt. % of the quaternized oligourethane of any of claims 1 to 3.

5. A process for preparing a quaternized oligourethane having the general formula: 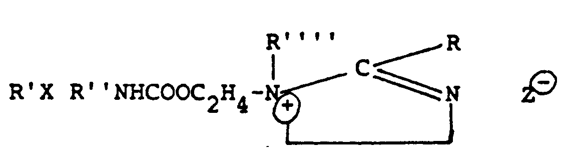 wherein X represents the radical -CONH-; R' is an ester resulting from the reaction of a C₂ - C₁₈ hydroxyalkanoic acid or a C₃ - C₁₈ hydroxyalkenoic acid and rosin; R is a C₁₁ - C₁₇ linear or branched alkyl, alkenyl, alkynyl or cycloalkyl; R'' is a C₆ - C₁₄ linear or branched alkyl, aryl, alkaryl, aralkyl or cycloalkyl or a dimer or trimer thereof; R'''' is hydrogen or a C₁ - C₄ linear or branched alkyl; and Z is R''''OSO₃, Cl, Br, I, NO₃, R'''''SO₃, (R'''')₃₋ₙHₙPO₄ or (R'''')₃₋ₙHₙPO₃
wherein R''''' is aryl, CH₧₃ or CF₃ and n is an integer of 0 to 3, which comprises the steps of:
(a) reacting a C₂-C₁₈ hydroxyalkanoic acid or a C₃-C₁₈ hydroxyalkenoic acid with rosin selected from wood rosin, gum rosin and tall oil rosin in the presence of an esterification catalyst;
(b) reacting the ester resulting from step (a) with a 1-hydroxyethyl-2- C₁₁-C₁₇ imidazoline and a C₆-C₁₄ polyisocyanate; and
(c) quaternizing the oligourethane resulting from step (b) by reacting it with a C₁-C₄ linear or branched alkyl halide, alkyl nitrate, alkyl phosphate, alkyl phosphite, alkyl sulfate, aryl sulfonate, alkyl sulfonate or a mineral acid or halogen acid.

6. The process of claim 5, wherein the acid is a C₆ - C₁₈ hydroxyalkanoic acid and the rosin is tall oil resin.

7. A process for preparing a quaternized oligourethane having the general formula: 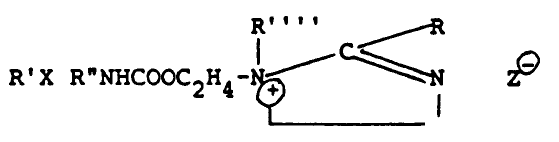 wherein X represents the radical -OCONH- R' is a C₃ - C₁₈ linear or branched alkyl, aryl, alkaryl, aralkyl, cycloalkyl or the radical 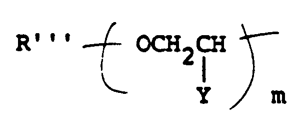 wherein Y is a hydrogen or methyl, R''' is a C₁ - C₆ alkyl and m is an integer of 1 to 20;
R is a C₁₁ - C₁₇ linear or branched alkyl, alkenyl, alkynyl or cycloalkyl;
R'' is a C₆ - C₁₄ linear or branched alkyl, aryl, alkaryl, aralkyl or cycloalkyl or a dimer or trimer thereof;
R'''' is hydrogen or a C₁ - C₄ linear or branched alkyl;
and
Z is R''''OSO₃, Cl, Br, I, NO₃, R'''''SO₃,
(R'''')₃₋ₙHₙPO₄ or (R'''')₃₋ₙHₙPO₃ wherein R''''' is aryl, CH₃ or CF₃, which comprises the steps of:
(a) reacting a C₃ - C₁₈ linear or branched alkyl, aryl, alkaryl, aralkyl, cycloalkyl, monofunctional alcohol or a monofunctional alcohol containing the radical 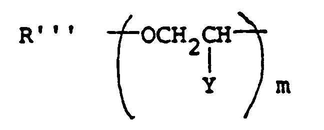 with a 1-hydroxyethyl-2- C₁₁-C₁₇ imidazoline and a C₆-C₁₄ polyisocyanate; and
(b) quaternizing the oligourethane resulting from step (a) by reacting it with a C₁ - C₄ linear or branched alkyl halide, alkyl nitrate, alkyl phosphate, alkyl phosphite, alkyl sulfate, aryl sulfonate, alkyl sulfonate or a mineral acid or halogen acid.

8. The process of claim 7 wherein the alcohol employed in step (a) is selected from the group consisting of hydroabietyl alcohol, butoxytriglycol and isostearyl alcohol.

## Claims (Claims for the following Contracting State(s): ES)

1. A printing ink comprising pigment dispersed in an ink vehicle comprised of a liquid petroleum hydrocarbon solution of an ink, resin and 1-20 wt. % of a quaternized oligourethane having the general formula: 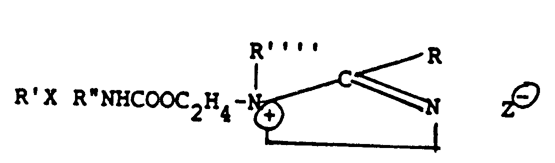 wherein X represents the radical -CONH- or -OCONH- such that when X is -CONH-, R' is an ester resulting from the reaction of a C₂-C₁₈ hydroxyalkanoic acid or a C₃-C₁₈ hydroxyalkenoic acid and rosin selected from wood rosin, gum rosin and tall oil rosin, and when X is -OCONH-, R' is a C₃-C₁₈ linear or branched alkyl, aryl, alkaryl, aralkyl, cycloalkyl or the radical 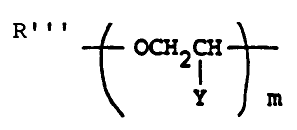 wherein Y is a hydrogen or methyl, R''' is a C₁ - C₆ alkyl and m is an integer of 1 to 20;
R is a C₁₁ - C₁₇ linear or branched alkyl, alkenyl, alkynyl or cycloalkyl;
R'' is a C₆ - C₁₄ linear or branched alkyl, aryl, alkaryl, aralkyl or cycloalkyl or a dimer or trimer thereof;
R'''' is hydrogen or a C₁ - C₄ linear or branched alkyl;
and
Z is R''''OSO₃, Cl, Br, I, NO₃, R'''''SO₃,
(R'''')₃₋ₙHₙPO₄ or (R'''')₃₋ₙHₙPO₃
wherein n is an integer of 0 to 3 and R''''' is aryl, CH₃ or CF₃.

2. The printing ink according to claim 1 wherein the rosin is tall oil rosin.

3. The printing ink according to claim 1 or claim 2 wherein X is the radical -OCONH-.

4. A process for preparing a quaternized oligourethane having the general formula: 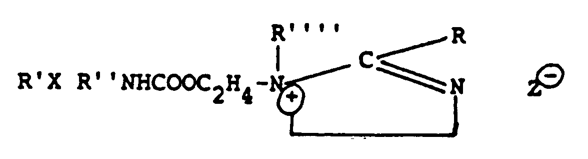 wherein X represents the radical -CONH-; R' is an ester resulting from the reaction of a C₂ - C₁₈ hydroxyalkanoic acid or a C₃ - C₁₈ hydroxyalkenoic acid and rosin; R is a C₁₁ - C₁₇ linear or branched alkyl, alkenyl, alkynyl or cycloalkyl; R'' is a C₆ - C₁₄ linear or branched alkyl, aryl, alkaryl, aralkyl or cycloalkyl or a dimer or trimer thereof; R'''' is hydrogen or a C₁ - C₄ linear or branched alkyl; and Z is R''''OSO₃, Cl, Br, I, NO₃, R''''SO₃, (R'''')₃₋ₙHₙPO₄ or (R'''')₃₋ₙHₙPO₃
wherein R''''' is aryl, CH₃ or CF₃ and n is an integer of 0 to 3, which comprises the steps of:
(a) reacting a C₂-C₁₈ hydroxyalkanoic acid or a C₃-C₁₈ hydroxyalkenoic acid with rosin selected from wood rosin, gum rosin and tall oil rosin in the presence of an esterification catalyst;
(b) reacting the ester resulting from step (a) with a 1-hydroxyethyl-2- C₁₁-C₁₇ imidazoline and a C₆-C₁₄ polyisocyanate; and
(c) quaternizing the oliguorethane resulting from step (b) by reacting it with a C₁-C₄ linear or branched alkyl halide, alkyl nitrate, alkyl phosphate, alkyl phosphite, alkyl sulfate, aryl sulfonate, alkyl sulfonate or a mineral acid or halogen acid.

5. The process of claim 4, wherein the acid is a C₆-C₁₈ hydroxyalkanoic acid and the rosin is tall oil rosin.

6. A process for preparing a quaternized oligourethane having the general formula: 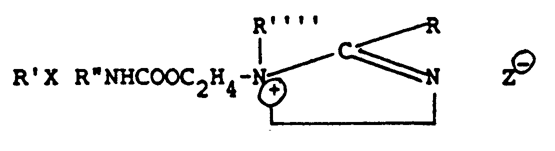 wherein X represents the radical -OCONH-; R' is a C₃ - C₁₈ linear or branched alkyl, aryl, aralkyl, cycloalkyl or the radical 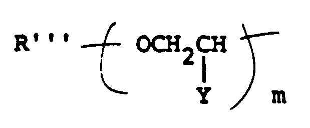 wherein Y is a hydrogen or methyl, R''' is a C₁ - C₆ alkyl and m is an integer of 1 to 20;
R is a C₁₁ - C₁₇ linear or branched alkyl, alkenyl, alkynyl or cycloalkyl;
R'' is a C₆ - C₁₄ linear or branched alkyl, aryl, alkaryl, aralkyl or cycloalkyl or a dimer or trimer thereof;
R'''' is hydrogen or a C₁ - C₄ linear or branched alkyl;
and
Z is R''''OSO₃, Cl, Br, I, NO₃, R'''''SO₃,
(R'''')₃₋ₙHₙPO₄ or (R'''')₃₋ₙHₙPO₃ wherein R''''' is aryl, CH₃ or CF₃, which comprises the steps of:
(a) reacting a C₃ - C₁₈ linear or branched alkyl, aryl, alkaryl, aralkyl, cycloalkyl mononofunctional alcohol or a monofunctional alcohol containing the radical 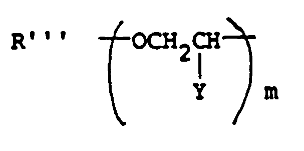 with a 1-hydroxyethyl-2- C₁₁-C₁₇ imidazoline and a C₆-C₁₄ polyisocyanate; and
(b) quaternizing the oligourethane resulting from step (a) by reacting it with a C₁ - C₄ linear or branched alkyl halide, alkyl nitrate, alkyl phosphate, alkyl phosphite, alkyl sulfate, aryl sulfonate, alkyl sulfonate or a mineral acid or halogen acid.

7. The process of claim 6 wherein the alcohol employed in step (a) is selected from the group consisting of hydroabietyl alcohol, butoxytriglycol and isostearyl alcohol.

## Patentansprüche (Patentansprüche für folgende(n) Vertragsstaat(en): AT, BE, CH, DE, FR, GB, GR, IT, LI, LU, NL, SE)

1. Ein quaternisiertes Oligourethan mit der allgemeinen Formel: 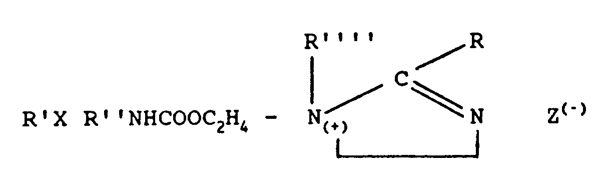 wobei X das Radikal -CONH- oder -OCONH- repräsentiert, derart, daß, wenn X gleich -CONH- ist, R' ein Ester resultierend aus der Reaktion von C₂-C₁₈ hydroxyalkanoischer Säure oder C₃-C₁₈ hydroxyalkenoischer Säure und Harz, ausgesucht aus Holzharz, Gummiharz oder dünnem Ölharz ist, und wenn X gleich -OCONH- ist, ist R' ein C₃-C₁₈ lineares oder verzweigtes Alkyl, Aryl, Alkaryl, Aralkyl, Cycloalkyl oder das Radikal 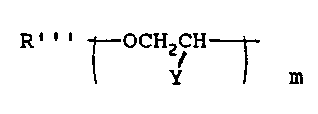 wobei Y ein Wasserstoff oder Methyl ist, R''' ein C₁-C₆ Alkyl und m eine ganze Zahl von 1-20 ist;
R ist ein C₁₁ bis C₁₇ lineares oder verzweigtes Alkyl, Alkenyl, Alkynyl oder Cycloalkyl;
R'' ist ein C₆ - C₁₄ lineares oder verzweigtes Alkyl, Aryl, Alkaryl, Aralkyl oder Cycloalkyl oder ein Dimer oder Trimer davon;
R'''' ist ein Wasserstoff oder ein C₁ - C₄ linear oder verzweigtes Alkyl; und
Z ist R''''OSO₃, R'''''SO₃, Cl, Br, I, NO₃ oder (R'''')₃₋ₙHₙPO₄ oder (R'''')₃₋ₙHₙPO₃
wobei n ist eine ganze Zahl von 0 bis 3, und R''''' ist Aryl, CH₃ oder CF₃.

2. Das quaternisierte Oligourethan von Anspruch 1, dadurch gekennzeichnet, daß das Harz ein dünnes Harzöl ist.

3. Ein quaternisiertes Oligourethan gemäß Anspruch 1, dadurch gekennzeichnet, daß X das Radikal -OCONH- ist.

4. Eine Druckfarbe beinhaltend ein dispergiertes Pigment in einem Farbträger bestehend aus einer flüssigen Petroleumkohlenwasserstofflösung einer Tinte, Harz und 1-20 Gew.% quaternisiertes Oligourethan von einem der Ansprüche 1-3.

5. Ein Verfahren zur Herstellung von quaternisiertem Oligourethan mit der allgemeinen Formel: 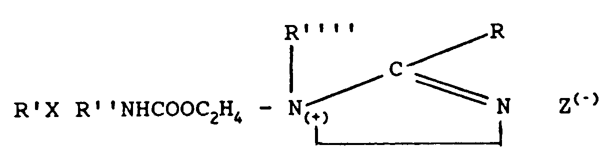 wobei X das Radikal -CONH- repräsentiert; R' ein Ester gebildet aus der Reaktion einer C₂-C₁₈-hydroxyalkanoischen Säure oder C₃-C₁₈-hydroxyalkenoischen Säure und Harz ist; R ist ein C₁₁-C₁₇ lineares oder verzweigtes Alkyl, Alkenyl, Alkynyl oder Cycloalkyl; R'' ist ein C₆-C₁₄ lineares oder verzweigtes Alkyl, Aryl, Alkaryl, Aralkyl oder Cycloalkyl oder ein Dimer oder Trimer hiervon; R'''' ist Wasserstoff oder ein C₁-C₄ linear oder verzweigtes Alkyl; und Z ist R''''OSO₃, Cl, Br, I, NO₃, R'''''SO₃, (R'''')₃₋ₙHₙPO₄ oder (R'''')₃₋ₙHₙPO₃, wobei R''''' Aryl, CH₃ oder CF₃ ist, und n eine ganze Zahl von 0-3, welches folgende Schritte beinhaltet:
(a) Reaktion von C₂-C₁₈ hydroxyalkanoischer Säure oder C₃-C₁₈ hydroxyalkenoischer Säure mit Harz ausgewählt aus Holzharz, Gummiharz und dünnem Ölharz in Anwesenheit eines Veresterungskatalysators;
(b) Reaktion des resultierenden Ester aus Schritt (A) mit einem 1-Hydroxyethyl-2-C₁₁-C₁₇ Imidazolin und einem C₆-C₁₄ Polyisocyanat; und
(c) Quaternisierung des resultierenden Oligourethans aus Schritt (b) durch Reaktion mit einem C₁-C₄ linearem oder verzweigtem Alkylhalogen, Alkylnitrat, Alkylphosphat, Alkylphospit, Alkylsulfat, Arylsulfonat, Alkylsulfonat oder einer Mineralsäure oder Halogensäure.

6. Das Verfahren von Anspruch 5, dadurch gekennzeichnet, daß die Säure eine C₆-C₁₈ hydroxyalkanoische Säure und das Harz dünnes Harzöl ist.

7. Ein Verfahren zur Herstellung eines quaternisierten Oligourethans mit der allgemeinen Formel 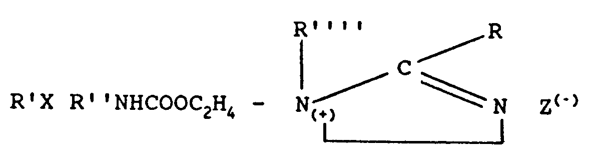 wobei X das Radikal -OCONH- repräsentiert, R' ein C₃-C₁₈ lineares oder verzweigtes Alkyl, Aryl, Alkaryl, Aralkyl, Cycloalkyl oder das Radikal 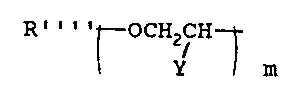 ist, wobei Y ein Wasserstoff oder Methyl ist, R''' ein C₁-C₆ Alkyl ist und m eine ganze Zahl von 1-20 ist;
R ist ein C₁₁-C₁₇ lineares oder verzweigtes Alkyl, Alkenyl, Alkynyl oder Cycloalkyl;
R'' ist ein C₆-C₁₄ lineares oder verzweigtes Alkyl, Aryl, Alkaryl, Aralkyl oder Cycloalkyl oder ein Dimer oder ein Trimer hiervon;
R'''' ist ein Wasserstoff oder ein C₁-C₄ lineares oder verzweigtes Alkyl; und
Z ist R''''OSO₃, Cl, Br, I, NO₃, R'''''SO₃,
(R'''')₃₋ₙHₙPO₄ oder (R'''')₃₋ₙHₙPO₃, wobei R''''' ein Aryl, CH₃ oder CF₃ ist, welcher folgende Schritte beinhaltet:
(a) Reaktion eines C₃-C₁₈ linear oder verzweigten Alkyl, Aryl, Alkaryl, Aralkyl, Cycloalkyl monofunktionalem Alkohol oder einem monofunktionalem Alkohol mit dem Radial 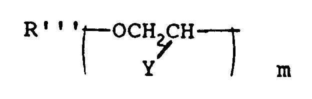 mit einem 1-Hydroxyethyl-2-C₁₁-C₁₇ Imidazolin und einem C₆-C₁₄ Polyisocyanat; und
(b) Quaternisierung des resultierenden Oligourethans aus Schritt (a) durch Reaktion mit einem C₁-C₄ linearem oder verzweigtem Alkylhalogenid, Alkylnitrat, Alkylphosphat, Alkylphosphit, Alkylsulfat, Arylsulfonat, Alkylsulfonat oder einer mineralischen Säure oder Halogensäure.

8. Das Verfahren aus Anspruch 7, wobei der in Schritt (a) verwendete Alkohol ausgewählt ist aus der Gruppe bestehend aus Hydroabietylalkohol, Butoxytriglycol und Isostearylalkohol.

## Patentansprüche (Patentansprüche für folgende(n) Vertragsstaat(en): ES)

1. Eine Druckfarbe beinhaltend ein dispergiertes Pigment in einem Farbträger bestehend aus einer flüssigen Petroleumkohlenwasserstofflösung einer Tinte, Harz und 1-20 Gew.% quaternisiertes Oligourethan mit der allgemeinen Formel: 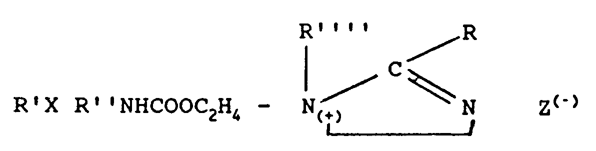 wobei X das Radikal -CONH- oder -OCONH- repräsentiert, derart, daß, wenn X gleich -CONH- ist, R' ein Ester resultierend aus der Reaktion von C₂-C₁₈ hydroxyalkanoischer Säure oder C₃-C₁₈ hydroxyalkenoischer Säure und Harz, ausgesucht aus Holzharz, Gummiharz oder dünnem Ölharz ist, und wenn X gleich -OCONH- ist, ist R' ein C₃-C₁₈ lineares oder verzweigtes Alkyl, Aryl, Alkaryl, Aralkyl, Cycloalkyl oder das Radikal 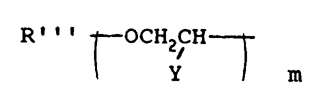 wobei Y ein Wasserstoff oder Methyl ist, R''' ein C₁-C₆ Alkyl und m eine ganze Zahl von 1-20 ist;
R ist ein C₁₁ bis C₁₇ lineares oder verzweigtes Alkyl, Alkenyl, Alkynyl oder Cycloalkyl;
R'' ist ein C₆ - C₁₄ lineares oder verzweigtes Alkyl, Aryl, Alkaryl, Aralkyl oder Cycloalkyl oder ein Dimer oder Trimer davon;
R'''' ist ein Wasserstoff oder ein C₁ - C₄ linear oder verzweigtes Alkyl; und
Z ist R''''OSO₃, R'''''SO₃, Cl, Br, I, NO₃ oder (R'''')₃₋ₙHₙPO₄ oder (R'''')₃₋ₙHₙPO₃
wobei n ist eine ganze Zahl von 0 bis 3, und R''''' ist Aryl, CH₃ oder CF₃.

2. Die Druckfarbe gemäß Anspruch 1, dadurch gekennzeichnet, daß das Harz ein dunnes Harzöl ist.

3. Die Druckfarbe gemäß Anspruch 1 oder Anspruch 2, dadurch gekennzeichnet, daß X das Radikal -OCONH- ist.

4. Ein Verfahren zur Herstellung von quaternisiertem Oligourethan mit der allgemeinen Formel: 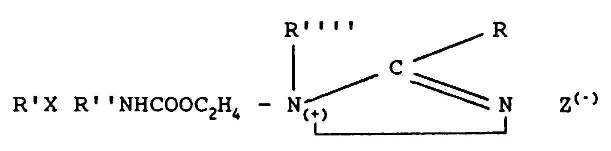 wobei X das Radikal -CONH- repräsentiert; R' ein Ester gebildet aus der Reaktion einer C₂-C₁₈-hydroxyalkanoischen Säure oder C₃-C₁₈-hydroxyalkenoischen Säure und Harz ist; R ist ein C₁₁-C₁₇ lineares oder verzweigtes Alkyl, Alkenyl, Alkynyl oder Cycloalkyl; R'' ist ein C₆-C₁₄ lineares oder verzweigtes Alkyl, Aryl, Alkaryl, Aralkyl oder Cycloalkyl oder ein Dimer oder Trimer hiervon; R'''' ist Wasserstoff oder ein C₁-C₄ linear oder verzweigtes Alkyl; und Z ist R''''OSO₃, Cl, Br, I, NO₃, R'''''SO₃, (R'''')₃₋ₙHₙPO₄ oder (R'''')₃₋ₙHₙPO₃, wobei R''''' Aryl, CH₃ oder CF₃ ist, und n eine ganze Zahl von 0-3, welches folgende Schritte beinhaltet:
(a) Reaktion von C₂-C₁₈ hydroxyalkanoischer Säure oder C₃-C₁₈ hydroxyalkenoischer Säure mit Harz ausgewählt aus Holzharz, Gummiharz und dünnem Ölharz in Anwesenheit eines Veresterungskatalysators;
(b) Reaktion des resultierenden Ester aus Schritt (A) mit einem 1-Hydroxyethyl-2-C₁₁-C₁₇ Imidazolin und einem C₆-C₁₄ Polyisocyanat; und
(c) Quaternisierung des resultierenden Oligourethans aus Schritt (b) durch Reaktion mit einem C₁-C₄ linearem oder verzweigtem Alkylhalogen, Alkylnitrat, Alkylphosphat, Alkylphospit, Alkylsulfat, Arylsulfonat, Alkylsulfonat oder einer Mineralsäure oder Halogensäure.

5. Das Verfahren von Anspruch 4, dadurch gekennzeichnet, daß die Säure eine C₆-C₁₈ hydroxyalkanoische Säure und das Harz dünnes Harzöl ist.

6. Ein Verfahren zur Herstellung eines quaternisierten Oligourethans mit der allgemeinen Formel 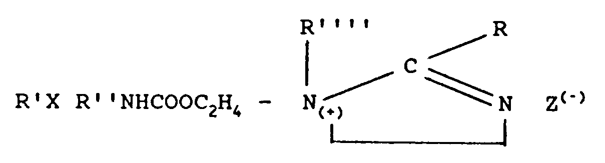 wobei X das Radikal -OCONH- repräsentiert, R' ein C₃-C₁₈ lineares oder verzweigtes Alkyl, Aryl, Alkaryl, Aralkyl, Cycloalkyl oder das Radikal 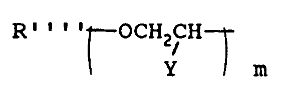 ist, wobei Y ein Wasserstoff oder Methyl ist, R''' ein C₁-C₆ Alkyl ist und m eine ganze Zahl von 1-20 ist;
R ist ein C₁₁-C₁₇ lineares oder verzweigtes Alkyl, Alkenyl, Alkynyl oder Cycloalkyl;
R'' ist ein C₆-C₁₄ lineares oder verzweigtes Alkyl, Aryl, Alkaryl, Aralkyl oder Cycloalkyl oder ein Dimer oder ein Trimer hiervon;
R'''' ist ein Wasserstoff oder ein C₁-C₄ lineares oder verzweigtes Alkyl; und
Z ist R''''OSO₃, Cl, Br, I, NO₃, R'''''SO₃,
(R'''')₃₋ₙHₙPO₄ oder (R'''')₃₋ₙHₙPO₃, wobei R''''' ein Aryl, CH₃ oder CF₃ ist, welcher folgende Schritte beinhaltet:
(a) Reaktion eines C₃-C₁₈ linear oder verzweigten Alkyl, Aryl, Alkaryl, Aralkyl, Cycloalkyl monofunktionalem Alkohol oder einem monofunktionalem Alkohol mit dem Radial 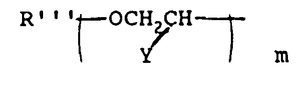 mit einem 1-Hydroxyethyl-2-C₁₁-C₁₇ Imidazolin und einem C₆-C₁₄ Polyisocyanat; und
(b) Quaternisierung des resultierenden Oligourethans aus Schritt (a) durch Reaktion mit einem C₁-C₄ linearem oder verzweigtem Alkylhalogenid, Alkylnitrat, Alkylphosphat, Alkylphosphit, Alkylsulfat, Arylsulfonat, Alkylsulfonat oder einer mineralischen Säure oder Halogensäure.

7. Das Verfahren aus Anspruch 6, wobei der in Schritt (a) verwendete Alkohol ausgewählt ist aus der Gruppe bestehend aus Hydroabietylalkohol, Butoxytriglycol und Isostearylalkohol.

## Revendications (Revendications pour l'(les) Etat(s) contractant(s) suivant(s): AT, BE, CH, DE, FR, GB, GR, IT, LI, LU, NL, SE)

1. Oligo-uréthanne quaternisé répondant à la formule générale 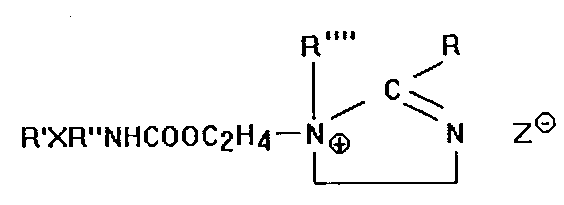 dans laquelle X représente un radical -CONH- ou -OCONH - tel que lorsque X est -CONH-, R' est un ester résultant de la réaction d'un acide hydroxyalcanoïque en C₂-C₁₈ ou d'un acide hydroxyalcénoïque en C₃-C₁₈ avec une colophane choisie parmi la colophane de bois, la colophane de gomme ou la colophane de tallol, et lorsque X est -OCONH-, R' est un groupe alkyle, aryle, alcaryle, aralkyle cycloalkyle linéaire ou ramifié en C₃-C₁₈ ou le radical 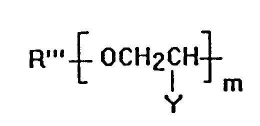 dans lequel Y est un atome d'hydrogène ou un groupe méthyle, R''' est un groupe alkyle en C₁-C₆ et m est un entier de 1 à 20 ;
R est est un groupe alkyle, alcényle, alcinyle ou cycloalkyle linéaire ou ramifié en C₁₁-C₁₈ ;
R'' est un groupe alkyle, aryle, alcaryle, aralkyle ou cycloalkyle linéaire ou ramifié en C₆-C₁₄ ou un dimère ou un trimère de ceux-ci ;
R'''' est un atome d'hydrogène ou un groupe alkyle linéaire ou ramifié en C₁-C₄ ; et
Z est R''''OSO₃, R'''''SO₃, Cl, Br, I, NO₃
(R'''')₃₋ₙHₙPO₄ ou (R'''')₃₋ₙHₙPO₃
où n est un entier de 0 à 3 et R''''' est un groupe aryle CH₃ ou CF₃.

2. Oligo-uréthanne quaternisé selon la revendication 1, dans lequel la colophane est une colophane de tallol.

3. Oligo-uréthanne quaternisé selon la revendication 1, dans lequel X est le radical -OCONH-.

4. Encre d'imprimerie comprenant un pigment dispersé dans un véhicule d'encre constitué d'une solution à'une encre dans un hydrocarbure de pétrole liquide, de résine et de 1 à 20 % en poids de l'oligo-uréthanne quaternisé selon l'une quelconque ds revendications 1 à 3.

5. Procédé de préparation d'un oligo-uréthanne quaternisé répondant à la formule générale 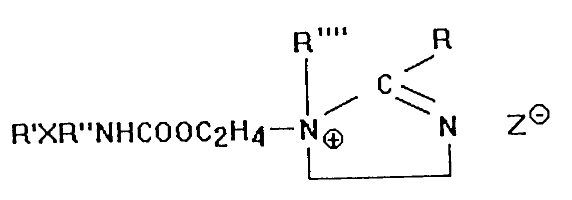 dans laquelle X représente un radical -CONH- ; R' est un ester résultant de la réaction d'un acide hydroxyalcanoïque en C₂-C₁₈ ou d'un acide hydroxyalcénoïque en C₃-C₁₈ et d'une colophane ; R est un groupe alkyle, alcényle, alcynyle ou cycloalkyle linéaire ou ramifié en C₁₁-C₁₇ ; R'' est un groupe alkyle, aryle, alcaryle, aralkyle ou cycloalkyle linéaire ou ramifié en C₆-C₁₄ ou un dimère ou un trimère de ceux-ci ; R'''' est un atome d'hydrogène ou un groupe alkyle linéaire ou ramifié en C₁-C₄ ; et Z est R''''OSO₃, Cl, Br, I, NO₃ (R''''')SO₃,(R'''')₃₋ₙHₙPO₄ ou (R'''')₃₋ₙHₙPO₃ dans lequel R''''' est un groupe aryle, CH₃ ou CF₃ et n est un entier de 0 à 3, que comprend les étapes consistant à .
(a) faire réagir un acide hydroxyalcanoïque en C₂-C₁₈ ou un acide hydroxyalcénoïque en C₃-C₁₈ avec une colophane choisie parmi la colophane de bois, la colophane de gomme et la colophane de tallol, en présence d'un catalyseur d'estérification ;
(b) faire réagir l'ester obtenu dans l'étape (a) avec une 1-hydroxyéthyl-2-(imidazoline en C₁₁-C₁₇) et un polyisocyanate en C₆-C₁₄ ;
(c) quaterniser l'oligo-uréthanne obtenu dans l'étape (b) en le faisant réagir avec un halogénure d'alkyle linéaire ou ramifié en C₁-C₄, un nitrate d'alkyle, un phosphate d'alkyle, un phosphite d'alkyle, un sulfate d'alkyle, un arylsulfonate, un alkylsulfonate ou un acide minéral ou un acide halogéné.

6. Procédé selon la revendication 5, dans lequel l'acide est un acide hydroxyalcanoïque en C₆-C₁₈ et la colophane est une résine de tallol.

7. Procédé de préparation d'un oligo-uréthanne quaternisé répondant à la formule générale 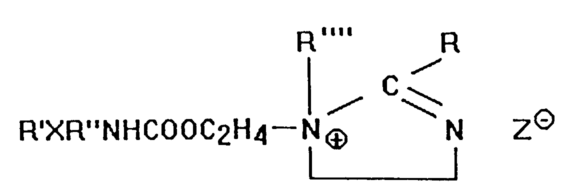 dans laquelle X représente un radical -OCONH -, R' est un groupe alkyle, aryle, alcaryle, aralkyle cycloalkyle linéaire ou ramifié en C₃-C₁₈ ou le radical 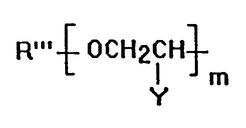 dans lequel Y est un atome d'hydrogène ou un groupe méthyle, R''' est un groupe alkyle en C₁-C₆ et m est un entier de 1 à 20 ;
R est un groupe alkyle, aryle, alkaryle, aralkyle cycloalkyle linéaire ou ramifié en C₁₁-C₁₇ ;
R'' est un groupe alkyle, aryle, alcaryle, aralkyle ou cycloalkyle linéaire ou ramifié en C₆-C₁₄ ou un dimère ou un trimère de ceux-ci.
R'''' est un atome d'hydrogène ou un groupe alkyle linéaire ou ramifié en C₁-C₄ ; et
Z et R''''OSO₃, Cl, Br, I, NO₃, R'''''SO₃, (R'''')₃₋ₙHₙPO₄ ou (R'''')₃₋ₙHₙPO₃ dans lequel R''''' est un groupe aryle, CH₃ ou CF₃, qui comprend les étapes consistant à :
(a) faire réagir un mono-alcool alkylique, arylique, alcarylique, aralkylique, cycloalkylique linéaire ou ramifié en C₃-C₁₈ ou un mono-alcool contenant le radical 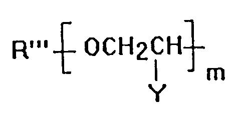 avec une 1-hydroxyéthyl-2-(imidazoline en C₁₁-C₁₇) et un polyisocyanate en C₆-C₁₄ ; et
(b) quaterniser l'oligo-uréthanne obtenu dans l'étape (a) en le faisant réagir avec un halogénure d'alkyle linéaire ou ramifié en C₁-C₄, un nitrate d'alkyle, un phosphate d'alkyle, un phosphite d'alkyle, un sulfate d'alkyle, un sulfonate d'aryle, un sulfonate d'alkyle, ou un acide minéral ou un acide halogéné.

8. Procédé selon la revendication 7, dans lequel l'alcool utilisé dans l'étape (a) est choisi parmi l'alcool hydroabiétylique, le butoxytriglycol et l'alcool isotéarylique.

## Revendications (Revendications pour l'(les) Etat(s) contractant(s) suivant(s): ES)

1. Encre d'imprimerie comprenant un pigment dispersé dans un véhicule d'encre constitué d'une solution, dans un hydrocarbure de pétrole liquide, d'une encre, d'une résine et de 1 à 20 % en poids d'un oligo-uréthanne quaternisé répondant à la formule générale : 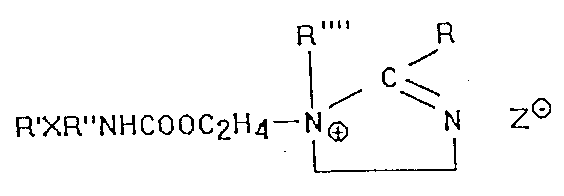 dans laquelle X représente un radical -CONH- ou -OCONH- tel que lorsque X est -CONH-, R' est un ester résultant de la réaction d'un acide hydroxyalcanoïque en C₂-C₁₈ ou d'un acide hydroxyalcénoïque en C₃-C₁₈ avec une colophane choisie parmi la colophane de bois, la colophane de gomme ou la colophane de tallol, et lorsque X est -OCONH-, R' est un groupe alkyle, aryle, alkaryle, aralkyle ou cycloalkyle linéaire ou ramifié en C₃-C₁₈, où le radical 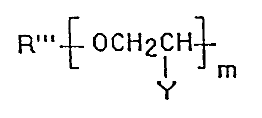 dans lequel Y est un atome d'hydrogène ou un groupe métyle, R''' est un groupe alkyle en C₁-C₆ et m est un entier de 1 à 20 ;
R est un groupe alkyle, alcényle, alcynyle ou cycloalkyle linéaire ou ramifié en C₁₁-C₁₇ ;
R'' est un groupe alkyle, aryle, alkaryle, aralkyle ou cycloalkyle linéaire ou ramifié en C₆-C₁₄ ou un dimère ou un trimère de ceux-ci ;
R'''' est un atome d'hydrogène ou un groupe alkyle linéaire ou ramifié en C₁-C₄ ; et
Z est R''''OSO₃, Cl, Br, I, NO₃, R'''''SO₃,
(R'''')₃₋ₙHₙPO₄ ou (R'''')₃₋ₙHₙPO₃
où n est un entier de 0 à 3 et R''''' est un groupe aryle, CH₃ ou CF₃.

2. Encre d'imprimerie selon la revendication 1, dans laquelle la colophane est la colophane de tallol.

3. Encre d'imprimerie selon la revendication 1 ou la revendication 2, dans laquelle X est le radical -OCONH-.

4. Procédé de préparation d'un oligo-uréthanne quaternisé répondant à la formule générale : 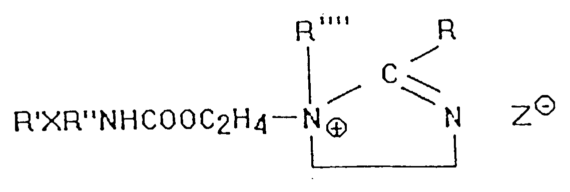 dans laquelle X représente un radical -CONH- ; R' est un ester résultant de la réaction d'un acide hydroxyalcanoïque en C₂-C₁₈ ou d'un acide hydroxyalcénoïque en C₃-C₁₈ et d'une colophane ; R est un groupe alkyle, alcényle, alcynyle ou cycloalkyle linéaire ou ramifié en C₁₁-C₁₇ ; R'' est un groupe alkyle, aryle, alkaryle, aralkyle ou cycloalkyle linéaire ou ramifié en C₆-C₁₄ ou un dimère ou un trimère de ceux-ci ; R'''' est un atome d'hydrogène ou un groupe alkyle linéaire ou ramifié en C₁-C₄ ; et Z est R''''OSO₃, Cl, Br, I, NO₃, (R''''') SO₃, (R'''')₃₋ₙHₙPO₄ ou (R'''')₃₋ₙHₙPO₃ dans lequel R''''' est un groupe aryle, CH₃ ou CF₃ et n est un entier de 0 à 3 ; ce procédé comprenant les étapes qui consistent à :
(a) faire réagir un acide hydroxyalcanoïque en C₂-C₁₈ ou un acide hydroxyalcénoïque en C₃-C₁₈ avec une colophane choisie parmi la colophane de bois, la colophane de gomme et la colophane de tallol, en présence d'un catalyseur d'estérification ;
(b) faire réagir l'ester obtenu dans l'étape (a) avec une 1-hydroxyéthyl-2-imidazoline C₁₁-C₁₇ et un polyisocyanate en C₆-C₁₄ ;
(c) quaterniser l'oligo-uréthanne obtenu dans l'étape (b) en le faisant réagir avec un halogénure d'alkyle, un nitrate d'alkyle, un phosphate d'alkyle, un phosphite d'alkyle, un sulfate d'alkyle, un arylsulfonate ou un alkylsulfonate linéaire ou ramifié en C₁-C₄, ou avec un acide minéral ou un acide halogéné.

5. Procédé selon la revendication 4, dans lequel l'acide est un acide hydroxyalcanoïque en C₆-C₁₈ et la colophane est une résine de tallol.

6. Procédé de préparation d'un oligo-uréthanne quaternisé répondant à la formule générale : 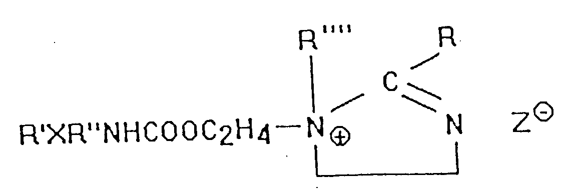 dans laquelle X représente un radical -OCONH-, R' est un groupe alkyle, aryle, alkaryle, aralkyle ou cycloalkyle linéaire ou ramifié en C₃-C₁₈, ou le radical 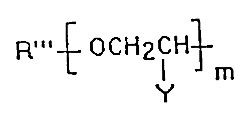 dans lequel Y est un atome d'hydrogène ou un groupe méthyle, R''' est un groupe alkyle en C₁-C₆ et m est un entier de 1 à 20 ;
R est un groupe alkyle, alcényle, alcynyle ou cycloalkyle linéaire ou ramifié en C₁₁-C₁₇ ;
R'' est un groupe alkyle, aryle, alkaryle, aralkyle ou cycloalkyle linéaire ou ramifié en C₆-C₁₄ ou un dimère ou un trimère de ceux-ci ;
R'''' est un atome d'hydrogène ou un groupe alkyle linéaire ou ramifié en C₁-C₄ ; et
Z est R''''OSO₃, Cl, Br, I, NO₃, R'''''SO₃, (R'''')₃₋ₙHₙPO₄ ou (R'''')₃₋ₙHₙPO₃ dans lequel R''''' est un groupe aryle, CH₃ ou CF₃ ; ce procédé comprenant les étapes qui consistent à :
(a) faire réagir un mono-alcool alkylique, arylique, alkarylique, aralkylique ou cycloalkylique linéaire ou ramifié en C₃-C₁₈, ou un mono-alcool contenant le radical 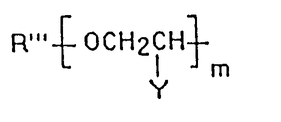 avec une 1-hydroxyéthyl-2-imidazoline en C₁₁-C₁₇ et un polyisocyanate en C₆-C₁₄ ; et
(b) quaterniser l'oligo-uréthanne obtenu dans l'étape (a) en le faisant réagir avec un halogénure d'alkyle, un nitrate d'alkyle, un phosphate d'alkyle, un phosphite d'alkyle, un sulfate d'alkyle, un sulfonate d'aryle ou un sulfonate d'alkyle linéaire ou ramifié en C₁-C₄, ou avec un acide minéral ou un acide halogéné.

7. Procédé selon la revendication 6, dans lequel l'alcool utilisé dans l'étape (a) est choisi parmi l'alcool hydroabiétylique, le butoxytriglycol et l'alcool isostéarylique.
